# EUROPEAN PATENT APPLICATION

(11) **EP 1 397 993 A1**
(43) Date of publication of application: **17.03.2004**
(21) Application number: 03255559.1
(22) Date of filing: 05.09.2003
(51) Int. Cl.: A61B 5/08, A61B 5/087

(54) **A rotary variable orifice valve**

(30) Priority: 11.09.2002 GB 0221045
(71) Applicant: Micro Medical Limited, Rochester, Kent ME1 2AZ (GB)
(72) Inventor: Lawson, Christopher Patrick, Rochester Kent ME1 2BN (GB)
(74) Representative: Jones, Graham H.

(57) **Abstract**

A rotary variable orifice valve (42) comprising a cylindrical member (46), a sleeve (52) which is a rotational fit with respect to the cylindrical member (46), and an orifice (44). In one embodiment, the cylindrical member (46) has an aperture (50), and the sleeve (52) has the orifice (44). The aperture (50) and the orifice (44) are positioned such that they overlap as the sleeve (52) is rotated by a motor (12).

## Description

This invention relates to a rotary variable orifice valve. The rotary variable orifice valve may be used in apparatus for measuring the strength of a person's respiratory muscles. The rotary variable orifice valve may also be used for other applications.

According to one non-limiting embodiment of the present invention there is provided a rotary variable orifice valve comprising a cylindrical member, a sleeve which is a rotational fit with respect to the cylindrical member, and an orifice.

Preferably, the sleeve is a rotational fit inside the cylindrical member. The sleeve may alternatively be a rotational fit over the cylindrical member.

The orifice may be of a shape that causes the resistance to flow of the rotary variable orifice valve to increase with rotation.

Preferably, the orifice is of a triangular shape. The orifice may be of other shapes if desired.

The rotary variable orifice valve may be one in which the cylindrical member has an aperture, the sleeve has the orifice, and the aperture and the orifice are positioned such that they overlap as the sleeve rotates. Alternatively, the rotary variable orifice valve may be one in which the cylindrical member has the orifice, the sleeve has the aperture, and the aperture and the orifice are positioned such that they overlap as the sleeve rotates. Alternatively, the rotary variable orifice valve may be one in which the orifice is positioned partly in the cylindrical member, and partly in the sleeve.

The present invention also extends to the combination of the rotary variable orifice valve, and a motor for operating the rotary variable orifice valve.

The rotary variable orifice valve may advantageously by employed in apparatus for measuring the strength of a person's respiratory muscles, which apparatus comprises a mouthpiece for the person, a flow transducer, a pressure transducer, a variable orifice valve, a motor for operating the variable orifice valve, and microprocessor control means, the microprocessor control means being such that it is able to control the motor to cause the variable orifice valve to vary its orifice size and thereby to maintain a constant predetermined pressure and enable the measurement of the flow rate generated by the person, or to maintain a constant predetermined flow rate and enable the measurement of the pressure generated by the person.

Usually, the flow rate or the pressure generated by the person will be generated by inhalation, but exhalation may be employed if desired.

Preferably, the apparatus of the invention will be used such that the microprocessor control means maintains different constant predetermined pressures, and measures the flow rate generated by the person at these constant predetermined pressures. If desired however, the apparatus of the present invention may be used such that the microprocessor control means maintains different predetermined flow rates and measures the pressure generated by the person. Either way, a maximum inspiratory pressure curve can be built up, and weak parts of the person's respiratory muscles can be seen from the curve. Corrective respiratory exercises can then be prescribed to strengthen any weak range or ranges of the respiratory muscles. For persons with weak respiratory muscles, the variable orifice will generally be small for the maximum inspired flow rate at a chosen pressure. For persons with strong respiratory muscles, the variable orifice will generally be large for the maximum inspired flow rate at a chosen pressure. Various exercises can be prescribed for persons with weak respiratory muscles over various ranges in order to improve the strength of the respiratory muscles over these ranges.

The apparatus may include a control circuit, the flow transducer being connected to the control circuit, the pressure transducer being connected to the variable orifice valve and to the control circuit, the pressure transducer being connected to the rotary variable orifice valve and to the control circuit, and the control circuit being connected to the microprocessor control means.

The microprocessor control means may comprise a microprocessor circuit, display means and a keypad.

The display means may be a display screen and/or a hard copy print device.

Preferably, the mouthpiece has a flange at the end of the mouthpiece that goes into the person's mouth. The flange helps the person's mouth to seal around the mouthpiece during the inhalation.

With the use of the rotary variable orifice valve, friction may be independent of applied pressure. The relationship between the resistance to flow and rotation of the valve is able easily to be adjusted by the shape of the orifice.

Embodiments of the invention will now be described solely by way of example and with reference to the accompanying drawings in which:
Figure 1 shows apparatus for measuring the strength of a person's respiratory muscles;
Figure 2 is a block circuit diagram of the apparatus shown in Figure 1;
Figure 3 shows a curve obtained by measuring pressure against flow and obtaining maximum inspired flows at different pressures;
Figure 4 is a perspective view of first rotary variable orifice valve which is for use in the apparatus shown in Figure 1;
Figure 5 is a perspective partially cut-away view of a second rotary variable orifice valve which is for use in the apparatus shown in Figure 1; and
Figure 6 is a view like Figure 5 but without being cut-away.

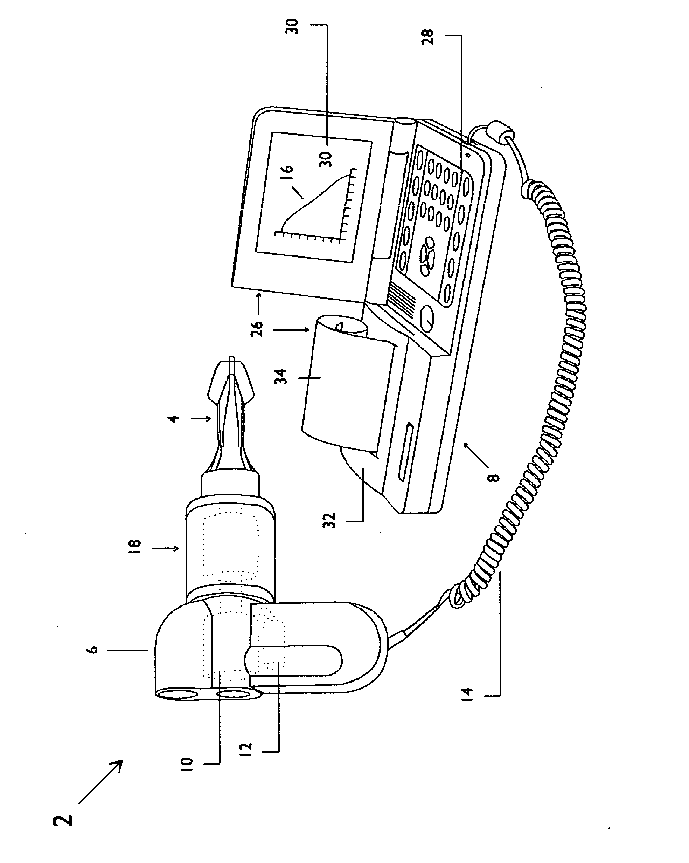

Referring to Figures 1 - 4, there is shown apparatus 2 for measuring the strength of a person's respiratory muscles. The apparatus 2 comprises a mouthpiece 4 for being inhaled through on by the person, a variable orifice valve arrangement 6, and microprocessor control means 8. The variable orifice valve arrangement 6 comprises a variable orifice valve 10 and a motor 12 for operating the variable orifice valve 10. The microprocessor control means 8 is such that it is able to control the motor 12 to cause the variable orifice valve 10 to vary its orifice size and thereby to maintain a constant predetermined pressure and enable the measurement of the flow rate generated by the person, or to maintain a constant predetermined flow rate and enable the measurement of the pressure generated by the person.

The variable orifice valve arrangement 6 also comprises a control circuit 20 and a pressure transducer 22. A flow transducer 18 is positioned between the mouthpiece 4 and the variable orifice valve arrangement 6.

The constant respiratory pressure transducer 6 is connected to the microprocessor control means 8 by a lead 14 as shown in Figure 1.

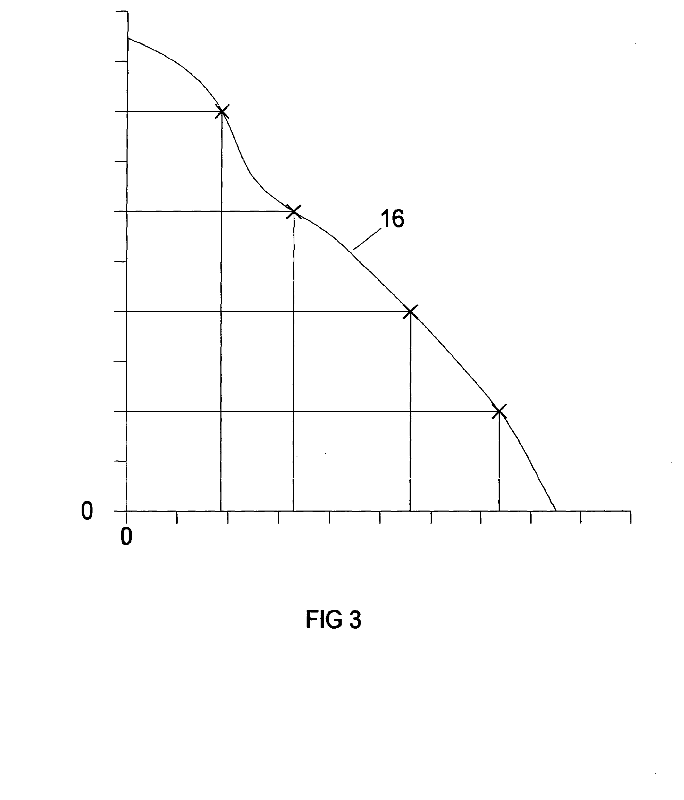

During use of the apparatus 2, for a person with weak lungs, the orifice in the variable orifice valve 10 will usually be relatively small for the maximum inspired flow rate. For a person with strong lungs, the orifice in the variable orifice valve 10 will usually be relatively large for the maximum inspired flow rate. Measurements can be taken of pressure against flow in order to build up a maximum inspiratory pressure curve 16 as shown in Figure 3. If the measurements being taken fluctuate due to uneven inhalation by the person, then a suitable algorithm may be employed to provide an average for each measurement. The curve 16 is then useful for identifying areas of weakness in the person's respiratory muscles. The person, for example a patient shortly to undergo major heart surgery, or an athlete, can then be given remedial exercises to strengthen their respiratory muscles over the weak range or ranges. In the case of persons about to undergo major surgery, the improved respiratory muscles will increase their chances of survival. In the case of athletes, improved respiratory muscles may result in improved performances.

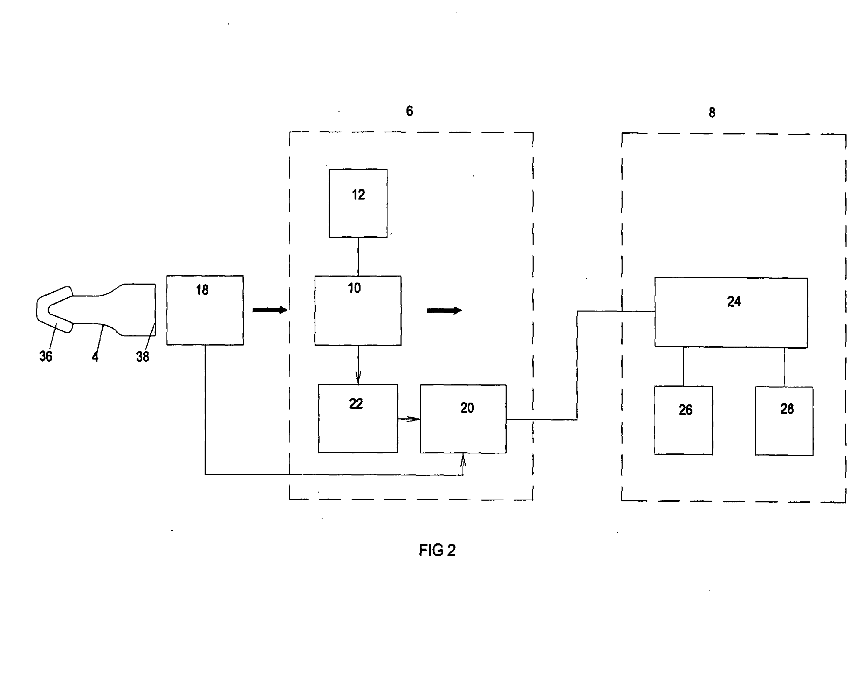

As shown in Figure 2, the flow transducer 18 is connected to the control circuit 20. The pressure transducer 22 is connected to the variable orifice valve 10 and to the control circuit 20. The control circuit 20 is connected to a microprocessor circuit 24 of the microprocessor control means 8. The microprocessor circuit 24 is also connected to display means 26 and a keypad 28.

As shown in Figure 1, the display means 26 comprises a display screen 30 and a hard copy print device 32. The display screen 30 is shown displaying a maximum inspiratory pressure curve 16. The print device 32 is shown having provided a hard copy print 34.

The mouthpiece 4 has a flange 36 at the end of the mouthpiece that goes into the person's mouth. The flange 36 helps the person's mouth to seal around the mouthpiece 4 during inhalation. The other end 38 of the mouthpiece 4 is cylindrical for being a push fit over a cylindrical part 40 of the constant respiratory pressure transducer 6.

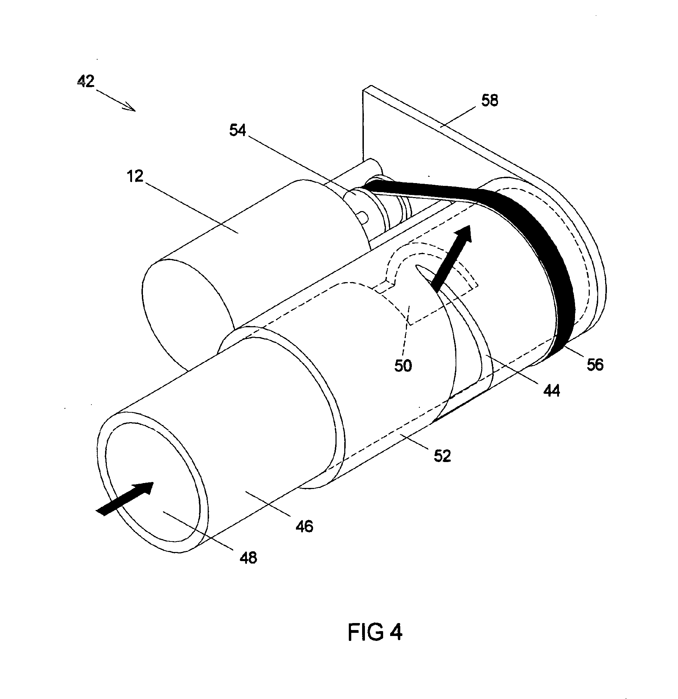

Figure 4 shows in detail the variable orifice valve 10 as a rotary variable orifice valve 42. The rotary variable orifice valve 42 does not generate substantial friction so that friction is substantially independent of applied pressure. The relationship between resistance to flow and rotation of the valve is easily adjusted by adjusting the shape of an orifice 44. The orifice 44 is of a shape that causes the resistance to flow of the rotary variable orifice valve 42 to increase with rotation. More specifically, the orifice 44 is of a triangular shape as shown.

The rotary variable orifice valve 42 comprises a cylindrical member 46 having a bore 48 and a rectangular aperture 50. The orifice 44 is in a sleeve 52 which is a rotational fit over the cylindrical member 46. As shown in Figure 4, the cylindrical member 46 is in the form of a short tube. During use of the rotary variable orifice valve 42, the sleeve 52 rotates over the cylindrical member 46, and the orifice 44 overlaps by varying amounts the rectangular aperture 50. In this way, the effective size of the orifice 44 is varied.

The rotation of the sleeve 52 is controlled by the motor 12. The motor 12 is mounted on one side of the sleeve 52. The motor 12 has a pulley 54 which drives an endless drive belt 56. The drive belt 56 is in frictional engagement with the outside of the sleeve 52 as shown. Thus rotation of the pulley 54 clockwise or anticlockwise, causes a corresponding rotation of the sleeve 52 via the drive belt 56.

The motor 12 is mounted on a motor mounting plate 58.

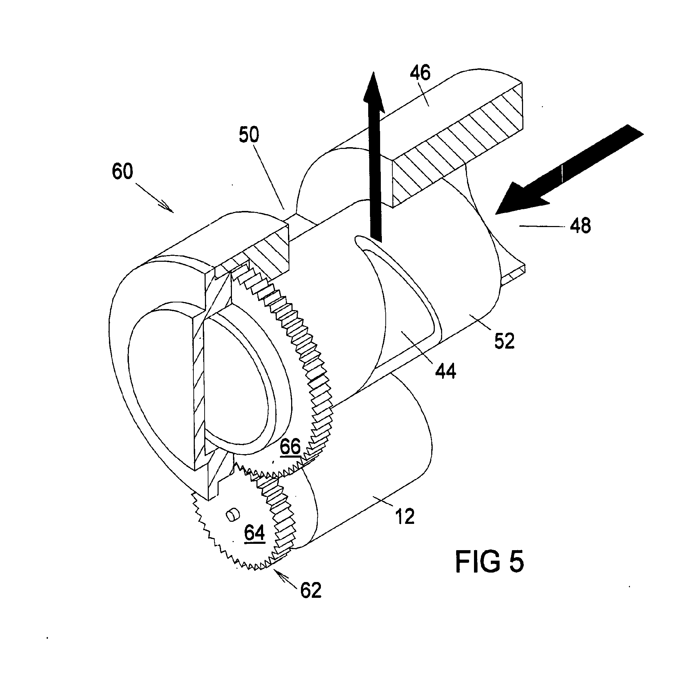

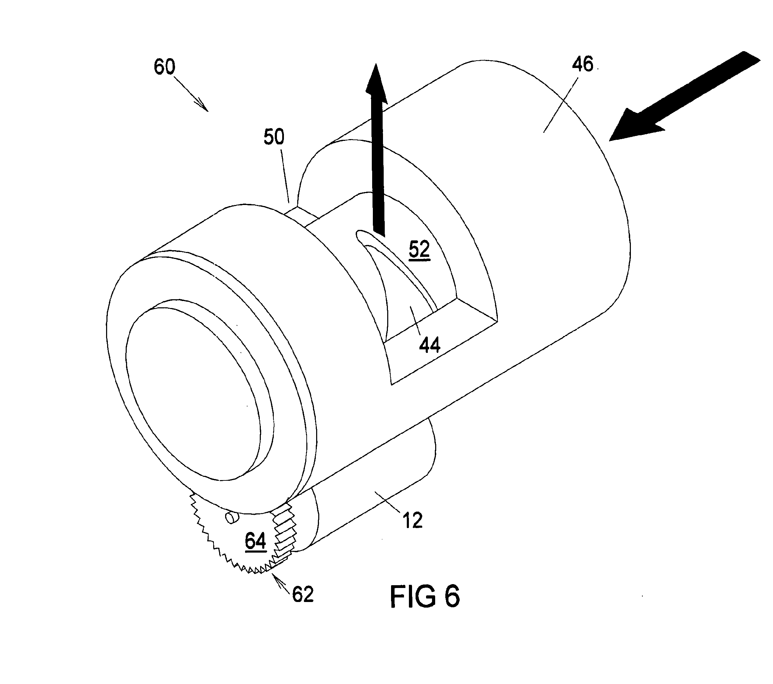

Referring now to Figures 5 and 6, there is shown a rotary variable orifice valve 60 which is an alternative to the rotary variable orifice valve 42 shown in Figure 4. Similar parts in the two rotary variable orifice valves 42, 60 have been given the same reference numerals for ease of comparison and understanding.

In the rotary variable orifice valve 60, it will be seen that the drive from the motor 12 is a gear drive 62 comprising two gears 64, 66 as shown. The gear drive 62 replaces the endless drive belt 56 shown in Figure 4. The gear drive 62 gives even less friction than the endless drive belt 56.

The rotary variable orifice valve 60 is such that the sleeve 52 is positioned inside the cylindrical member 46 and rotates inside the cylindrical member 46 by virtue of the gear drive 62. This arrangement reduces the moment of inertia of the rotating part in the form of the sleeve 52 as compared with the arrangement shown in Figure 4 in which the sleeve 52 is positioned over the tube 46. The reduction of the moment of inertia provides an increase of speed of response of the rotary variable orifice valve 60 as compared with the rotary variable orifice vale 42.

it is to be appreciated that the embodiments of the invention described above with reference to the accompanying drawings have been given by way of example only and that modifications may be effected. Thus, for example, the motor 12 may be arranged to drive the sleeve 56 by means other than the drive belt 56 or the gears 64, 66. If desired, the motor 12 could be mounted in line with the cylindrical member 46 and then connected to the sleeve 52 by an appropriate drive arrangement. The motor 10 may be battery operated and/or mains operated. In Figure 3, the curve 16 has been obtained by causing the microprocessor control means 8 to control the motor 12 to cause the variable orifice valve 10 to vary its orifice size and thereby to maintain constant predetermined pressures, so that the flow rate generated by the person can be measured. If desired however, the curve 16 may be obtained by causing the microprocessor control means 16 to control the motor 12 to cause the variable orifice valve 10 to vary its orifice size and thereby to maintain constant predetermined flow rates, so that the pressure generated by the person can be measured.

## Claims

1. A rotary variable orifice valve comprising a cylindrical member, a sleeve which is a rotational fit with respect to the cylindrical member, and an orifice.

2. A rotary variable orifice valve according to claim 1 in which the sleeve is a rotational fit inside the cylindrical member.

3. A rotary variable orifice valve according to claim 1 in which the sleeve is a rotational fit over the cylindrical member.

4. A rotary variable orifice valve according to any one of the preceding claims in which the orifice is of a shape that causes the resistance to flow of the rotary variable orifice valve to increase with rotation.

5. A rotary variable orifice valve according to any one of the preceding claims in which the orifice is of a triangular shape.

6. A rotary variable orifice valve according to any one of the preceding claims in which the cylindrical member has an aperture, the sleeve has the orifice, and the aperture and the orifice are positioned such that they overlap as the sleeve rotates.

7. A rotary variable orifice valve according to any one of claims 1 - 5 in which the cylindrical member has the orifice, the sleeve has an aperture, and the aperture and the orifice are positioned such that they overlap as the sleeve rotates.

8. A rotary variable orifice valve according to any one of claims 1 - 5 in which the orifice is positioned partly in the cylindrical member and partly in the sleeve.

9. The combination of a rotary variable orifice valve according to any one of the preceding claims, and a motor for operating the rotary variable orifice valve.
